(19) Europäisches Patentamt
European Patent Office
Office européen des brevets

(11) Publication number: **0 186 255 B1**

# (12) EUROPEAN PATENT SPECIFICATION

(45) Date of publication of patent specification: **10.04.91**

(51) Int. Cl.5: **C07C 37/16**, C07C 39/15, C07C 43/23, C07C 39/367

(21) Application number: **85301406.6**

(22) Date of filing: **28.02.85**

## (54) Process for preparing benzyl substituted phenols, dibenzylphenolic compounds, and the antioxidant use of such phenols.

(30) Priority: **24.12.84 US 685732**

(43) Date of publication of application:
**02.07.86 Bulletin 86/27**

(45) Publication of the grant of the patent:
**10.04.91 Bulletin 91/15**

(84) Designated Contracting States:
**BE CH DE FR GB LI NL**

(56) References cited:

**EP-A- 0 103 241**
**US-A- 3 833 672**
**US-A-33 679 81**
**US-A- 2 831 898**
**US-A- 4 514 577**
**US-A-38 165 44**

**J. Amer. Chem.Soc. 53, p. 2379-2382 (1931)**

**CHEMICAL ABSTRACTS, vol.78, no. 17, April 30, 1973, page 438, ref.no. 110754s; Columbus, Ohio, US; A. R. ABDURASULEVA et al, "Alkylation of phenols and their ethers in the presence of small amounts of catalysts. Benzylation and cycloalkylation of 4-cholorophenol and 4-chloroanisole"**

(73) Proprietor: **ETHYL CORPORATION**
**Ethyl Tower 451 Florida Boulevard**
**Baton Rouge Louisiana 70801(US)**

(72) Inventor: **Filbey, Allen Howard**
**13442 Anne Cleves Avenue**
**Baton Rouge, LA 70816(US)**
Inventor: **Braxton, Henry Green, Jr.**
**11888 Longridge Drive, Apt. 2049**
**Baton Rouge, LA 70816(US)**
Inventor: **Meltsner, Bernard Ralph**
**2711 Crooks Road**
**Royal Oak, MI 40783(US)**

(74) Representative: **Bizley, Richard Edward et al**
**BOULT, WADE & TENNANT 27 Furnival Street**
**London EC4A 1PQ(GB)**

EP 0 186 255 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 82, no. 17, April 28, 1975,page 455, ref.no. 111403w; Columbus, Ohio, US; V. P. LANIN et al, "State of high-molecular weight ortho-substituted phenols studied by a cryoscopic method"

PATENTS ABSTRACTS OF JAPAN, vol. 9, no. 45, February 26, 1985, page 1768, C-268 & JP-A-59 186 937

## Description

This invention is concerned with benzylation of phenols.

Ortho-alkylated phenols are valuable as antioxidants and chemical intermediates. One method of making them is by the reaction of an olefin with a phenol in the presence of an aluminium phenoxide catalyst (Ecke et al. U.S. 2,831,898, issued 22 April 1958). Phenols have also been alkylated by reaction with an olefin using an alumina catalyst (Napolitano U.S. 3,367,981, issued 6 February 1968). L.H. Klemm et al . report the ortho-alkylation of phenol with n -propanol using an alumina catalyst (J. Org. Chem. 45, pages 4320-6).

Much less seems to be known about the benzylation of phenols for which the above-mentioned prior art processes are unsuitable. W.J. Hickenbottom, J. Chem. Soc. , 80, pages 2844-9, report the preparation of 2-benzyl, 2,4-dibenzyl and 2,6-dibenzyl phenols by heating phenol with sodium hydroxide in toluene and reacting this with benzyl chloride.

R.C. Huston et al. , J. Am. Chem. Soc. 53 , page 2379, describe the reaction of benzyl alcohol with p -cresol using an aluminium chloride catalyst to make dibenzyl-p -cresol. However, no utility is suggested for this compound.

Brindell et al ., U.S. 3,816,544, issued 11 June 1974, disclose the reaction of 2,6-dibenzylphenol with formaldehyde to form 4,4'-methylenebis-(2,6-dibenzylphenol) but do not disclose any process for making 2,6-dibenzylphenol.

Starks U.S. 4,105,699, issued 8 August 1978, discloses the reaction of phenol and benzyl alcohol in the liquid phase using an alpha-alumina monohydrate catalyst to make mainly ortho-benzylphenol plus minor amounts of 2,6-dibenzylphenol.

In contrast, according to an aspect of the present invention, di-o -benzylated phenols may be made in high yield in a continuous process by passing a mixture of a phenol and a benzyl alcohol in the vapor phase through an activated alumina catalyst at about 225-240° C.

The 2,6-dibenzylphenols having certain 4 substituents are new compounds and show antioxidant activity in organic materials.

One aspect of the invention is a process for making di-ortho benzyl substituted phenols, said process comprising contacting a mixture of a phenol and a benzyl alcohol in the vapor phase with an activated gamma-alumina catalyst at a temperature in the range of 225-450° C, preferably 250-350° C said temperature being high enough to maintain said phenol and benzyl alcohol in the vapor phase at reaction conditions, said phenol having at least both positions ortho to its phenolic hydroxyl group unsubstituted except for hydrogen, and said mixture having a molar ratio of 1:1-3 of said phenol to said benzyl alcohol.

The process is applicable to a broad range of phenols. The term "a phenol" is used in a generic sense to include all aromatic hydroxy compounds having at least one hydroxy group bonded to an aromatic ring. The phenol must be capable of being heated to a temperature high enough to convert it to the vapor phase without excessive decomposition. Examples of typical phenols include phenol, p -cresol, 4-ethylphenol, 4-phenylphenol, β-naphthol, hydroquinone, 4-methoxyphenol, and 4-ethoxyphenol.

The novel 2,6-dibenzyl-4-substituted compounds of the invention may be prepared from a more preferred class of phenols having the structure

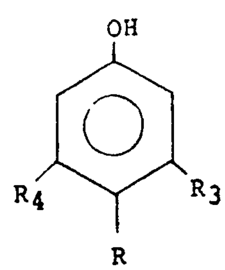

wherein $R_3$ and $R_4$ are independently selected from H, alkyl, cycloalkyl, halo, aralkyl, alkaryl, aryl, alkoxy, or alkenyl and wherein R is benzyl, cycloalkyl, hydroxy, alkoxy, alkenyl, or hydrocarbonyl.

Preferably $R_3$ and $R_4$ are selected from hydrogen, alkyls containing 1-20 carbon atoms, alkenyls containing 2-20 carbon atoms, cycloalkyls containing 5-8 carbon atoms, aryls containing 6-12 carbon atoms, halogens, hydroxy, alkoxys containing 1-4 carbon atoms, alkaryls of 7-12 carbon atoms and aralkyls of 7-12 carbon atoms. Also preferably, R is selected from alkenyls containing 2-20 carbon atoms, cycloalkyls containing 5-8 carbon atoms, hydroxy, alkoxys containing 1-4 carbon atoms, and hydrocarbonyls containing

1-20 carbon atoms.

Thus suitable starting materials for forming the novel 2,6-dibenzyl-4-substituted phenols include the following:

4-cyclopentylphenol, 4-cyclohexylphenol, and the like; 4-benzylphenol; 4-methoxyphenol, 4-ethoxyphenol, 4-propoxyphenol, 4-butoxyphenol, 4-isobutoxyphenol, and the like; 4-allylphenol; and 4-stearylphenol.

Of course all of the above described starting materials may also have 3-position and 5-position substituents in accordance with the definitions of $R_3$ and $R_4$ as given above or any other substituent which does not prevent formation of the 2,6-dibenzyl-4-substituted compounds. Thus the following are also suitable starting materials according to the invention: 3,5-dimethylphenol, 3,5-di-tert-butylphenol, 3-ethyl-5-isopropylphenol, 3-ethoxy-5-cyclohexylphenol, 3,4,5-tricyclohexylphenol, 3,5-diethyl-4-benzylphenol, 3,5-dimethyl-4-hydroxyphenol, 3,5-diethoxy-4-methoxyphenol, 3,5-dimethyl-4-ethoxyphenol, 3-methyl-4-ethoxy-5-cyclohexylphenol, 3,5-diisopropyl-4-allylphenol, and 3-chloro-4-stearyl-5-cyclohexylphenol.

The novel compounds of the invention include compounds having the structure

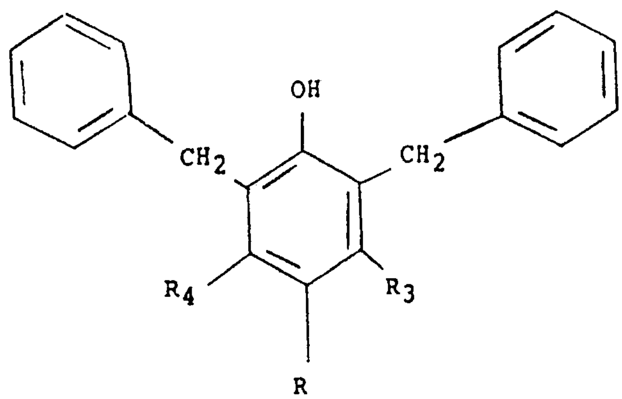

wherein $R_3$ and $R_4$ are independently H, alkyl, cycloalkyl, halo, aralkyl, alkaryl, aryl, alkoxy, or alkenyl and wherein R is benzyl, cycloalkyl, hydroxy, alkoxy, alkenyl, or hydrocarbonyl.

Thus the novel compounds of the invention include 2,6-dibenzyl-4-cyclohexylphenol, 2,6-dibenzyl-4-cyclopentylphenol; 2,4,6-tribenzylphenol; 2,6-dibenzyl-4-methoxyphenol, 2,6-dibenzyl-4-ethoxyphenol, 2,6-dibenzyl-4-isopropoxyphenol, 2,6-dibenzyl-4-butoxyphenol; 2,6-dibenzyl-4-allylphenol; 2,6-dibenzyl-4-stearylphenol wherein the stearyl group is $C_{17}H_{35}CO$.

Of course the classes of novel compounds of the invention are not limited to the compounds recited above but also include those compounds which have 3 and 5 substituents in accordance with the invention. Thus, the following compounds are also included in the invention: 2,6-dibenzyl-3,5-diethyl-4-cyclohexylphenol, 2,4,6-tribenzyl-3,5-dimethylphenol, 2,6-dibenzyl-3,5-diethoxy-4-hydroxyphenol, 2,6-dibenzyl-3,5-diethyl-4-methoxyphenol, 2,6-dibenzyl-3-ethyl-4-allyl-5-cyclohexylphenol, and 2,6-dibenzyl-3,5-diethyl-4-stearylphenol.

More preferred among the novel compounds of the invention are the 2,6-dibenzyl-4-substituted phenols wherein $R_3$ and $R_4$ are H. Still more preferred are those 2,6-dibenzylphenols of the invention wherein the 4 substituent is cycloalkyl wherein the $R_3$ and $R_4$ substituents are H. A highly preferred compound of the invention is the compound 2,4,6-tribenzylphenol which is available as a natural product of the benzylation process described herein and may be prepared from phenol.

A preferred class of reactant phenols for the process of the invention contains the structure

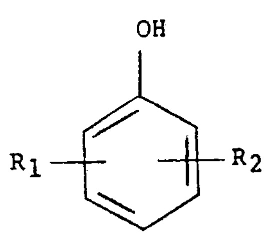

wherein $R_1$ and $R_2$ are selected from the group consisting of hydrogen, alkyl containing 1-20 carbon atoms, alkenyl containing 2-20 carbon atoms, cycloalkyl containing 5-8 carbon atoms, aryl containing 6-12 carbon

atoms, halogen, hydroxy, and $C_{1-4}$ alkoxy.

These include 4-sec -eicosylphenol, 4-allylphenol, 4-stearylphenol, 4-cyclohexylphenol, 4-methoxyphenol, 4-ethoxyphenol, 4-propoxyphenol, 4-butoxyphenol, 4-isobutoxyphenol, and hydroquinone.

Highly preferred reactants in the process are the compound phenol, $C_6H_5OH$, and mono lower alkyl derivatives thereof such as p -cresol, p -ethylphenol, and p -n -butylphenol. The most preferred phenol reactant is the compound phenol.

Benzyl alcohols are the class of compounds which have a hydroxymethyl group bonded to a benzene ring. The benzene ring may be unsubstituted or may be substituted with groups such as alkyl, halogen, and alkoxy. Typical benzyl alcohols include p -methylbenzyl alcohol, p -ethylbenzyl alcohol, o -methylbenzyl alcohol, p -isobutylbenzyl alcohol, p -chlorobenzyl alcohol, 2,4-dichlorobenzyl alcohol, o -bromobenzyl alcohol, p -methoxybenzyl alcohol, and p -ethoxybenzyl alcohol. The most preferred benzylating agent is the compound benzyl alcohol, $C_6H_5CH_2OH$.

The phenol reactant and benzyl alcohol reactant may be used in a wide mole ratio range such as 1-3 moles of the benzyl alcohol per mole of the particular phenol. It has been found that it is not beneficial to use much excess benzyl alcohol because it tends to react with itself to form dibenzyl ethers.

The reactant ratio when dibenzylation is desired is 1-3 moles of the benzyl alcohol reactant per mole of the phenolic reactant. When reacting the compound benzyl alcohol with the compound phenol, it has been found that high yields of 2,6-dibenzylphenol require a ratio of 1.5-2.5 moles of benzyl alcohol per mole of phenol.

Although a broad range of activated aluminas may be used, they are not all equivalent in performance. According to the invention an activated gamma-alumina catalyst is used (Alumina Properties , Russel et al ., published by Aluminum Company of America, 1956).

This alumina gives exceptionally high yields in the vapor phase reaction and exhibits an extremely low deactivation rate.

The process is carried out by placing an alumina catalyst bed in a suitable container and heating the catalyst bed to the desired temperature. This temperature should be high enough to maintain both the phenolic reactant and benzyl alcohol reactant in the vapor phase under the particular conditions such as pressure. The reaction is preferably conducted at close to atmospheric pressure although higher and lower pressures may be used. The temperature should also be high enough to cause the benzylation to proceed at a reasonable rate, but not so high as to cause decomposition. A useful range in which to experiment is 225-450 °C. Very good results have been achieved in the case of the compounds phenol and benzyl alcohol at temperatures of 250-450 °C.

While the catalyst bed is being heated to reaction temperature, it can be purged with an inert gas such as nitrogen. This will prevent oxidation of the reactants once chemical feed is started.

The phenolic and benzyl alcohol can be fed to the heated catalyst in separate feeds. An easy way to control the mole ratio is to first mix the phenolic reactant and benzyl alcohol reactant in the desired mole ratio and then feed the mixture to the heated catalyst. The mixture is preferably heated such that it provides a liquid feed.

The liquid feed can be fed directly to the catalyst bed. Preferably, the liquid feed is first passed through a pre-heater which rapidly heats the liquid feed to form a vapor mixture. The vapor is then passed through the catalyst bed.

Only a short contact time of the vapor mixture with the catalyst is required. Contact times of one second to 10 minutes are effective. A more preferred contact time is 5 seconds to 5 minutes. A still more preferred contact time is 5 seconds to one minute.

After traversing the catalyst bed, the effluent product is cooled to condense all vapors. After the water formed in the reaction is removed, the crude product may then be distilled to recover the desired product(s) and unconverted starting material.

The following Examples serve the better to illustrate the invention, Examples 1,2,3 and 10 being for comparison purposes only.

Comparative EXAMPLE 1

A continuous catalytic reactor was made by placing a pelleted gamma alumina (Harshaw 3438 T gamma alumina) in a quartz tube about 30 cm length and 2.5 cm in diameter. The 5 cm catalyst plug (approximately 16 g) was held in place between 5 cm of glass beads at the bottom of the tube and glass beads above the catalyst up to the top of the tube. Temperature in the catalyst was measured using a thermocouple probe. The top glass bead section functions as a pre-heater and had a separate electrical

heater. The catalyst section was heated by a separate clam-shell type electric heater. The top of the tube was fitted with a nitrogen inlet and a dropping funnel. The bottom connected through an air cooled condenser to a glass receiver.

In the initial run the catalyst bed was heated to 330-340 °C while purging the system with nitrogen. Then a 6:1 mole ratio mixture of benzyl alcohol and phenol was fed dropwise at the top of the tube at about 0.2 ml per minute. This was vaporized in the pre-heater section and the vapors passed downward through the gamma alumina catalyst. The products passed through the air cooled condenser and were collected in the receiver.

The major components in the effluent by VPC analysis were:

| | Percent |
|---|---|
| 2,6-dibenzylphenol | 18.9 |
| 2,4,6-tribenzylphenol | 6.8 |
| benzyl alcohol | 25.2 |
| dibenzyl ether | 15.2 |
| benzaldehyde | 11.4 |
| light unknown | 16.8 |

EXAMPLES 2-6

These Examples were conducted in the same manner as Example 1 except for reactant ratio and temperature. The following table gives the reaction conditions:

| Example | Temperature °C | Benzyl Alcohol to Phenol Mole Ratio | Feed Time (hrs.) | Total Feed (g.) |
|---|---|---|---|---|
| 2* | 275-340 | 6:1 | 1.25 | 42 |
| 3* | 290-300 | 6:1 | 5 | 84 |
| 4 | 290-300 | 3:1 | 3 | 87 |
| 5 | 300-320 | 2.5:1 | 6 | 136 |
| 6 | 280-290 | 2:1 | 33 | 585 |

* for comparison.

| | Example | | | | |
|---|---|---|---|---|---|
| Product | 2[1] | 3[2] | 4[2] | 5[2] | 6[2] |
| o-monobenzyl-phenol | 0.7-1.4 | 0.6-1.3 | 1-3.2 | 0.4-1 | 6.2-3.8 |
| 2,6-dibenzyl-phenol | 18.9-13.0 | 12-14.4 | 49-41 | 67.7-66.9 | 75-77 |
| tribenzylphenol | 6.8-7.3 | 5.4-8.0 | 16.3-13 | 18.9-19.3 | 11-14 |
| benzyl alcohol | 25.2-48.1 | 32.7-41.2 | 11.7-19.9 | 2.1-2.2 | |
| dibenzyl ether | 15.2-16.3 | 29-17.3 | 6.8-5.5 | 0.6-0.6 | |
| light unknown[3] | 16.8-2.8 | 7.8-5 | 7.7-2 | 0.2-2.4 | |

1 The first value is at the first stage of the reaction at 330-340°C and the second value is at the final stage of the reaction at 275-285°C.

2 The first value is at the start of the process and the second is towards the end.

3 Probably toluene.

These results show the critical sensitivity of dibenzylphenol yield on the benzyl alcohol-phenol mole ratio. At 6:1, less than 20 percent of the effluent was the desired dibenzylphenol. At 3:1, almost half of the product was dibenzylphenol and at 2:1, three-quarters of the effluent was dibenzylphenol.

EXAMPLES 7-8

Two more experiments were conducted at still lower mole ratios. Both were conducted at 280-290° C using 16 g Harshaw H-3438 T gamma alumina.

| Example | Mole Ratio | Total Time | Total Feed | Percent Benzylphenol o-Mono | 2,6-Di | Tri |
|---|---|---|---|---|---|---|
| 7 | 1.75:1 | 7 hr. | 88 g | 4.5-8 | 68-73.7 | 20-11 |
| 8 | 1.5:1 | 10.5 hr. | 97 g | 8.2-13 | 74.5-64 | 11-10.9 |

EXAMPLE 9

This experiment was conducted to measure the decay rate of catalytic activity. The same Harshaw H-3438 T gamma alumina catalyst (16 g) was used. The reaction zone was maintained at 280-290° C. Over a 33 hour period, 2.7 Kg of a 2:1 mole mixture of benzyl alcohol:phenol was passed through the catalyst in the vapor phase. Feed rate was varied during the course of the reaction to determine the effect of contact time on conversion. The composition of the product was as follows (VPC):

| | Initial | Mid-Point | End |
|---|---|---|---|
| Phenol | 2.6 | 4.1 | 4.3 |
| Benzyl alcohol | - | 3.3 | 4.6 |
| 2-Benzylphenol | 6.6 | 6.0 | 6.5 |
| 2,6-Dibenzylphenol | 72.3 | 64.6 | 66.2 |
| 2,4,6-tribenzylphenol | 13.9 | 16.0 | 11.7 |

The highest conversion to 2,6-dibenzylphenol was at a feed rate of 16 g hr. (76 percent) although even at a much higher feed rate of 56 g hr. the product was 65.1 percent 2,6-dibenzylphenol. From this it can be seen that the process is capable of very high production rates.

An experiment was carried out by reacting benzyl alcohol with phenol in the liquid phase using a gamma-alumina catalyst for comparative purposes.

COMPARATIVE EXAMPLE 10

In a reaction vessel was placed 47 g (0.5 mole) phenol, 75.6 g (1.2 moles) benzyl alcohol and 7.5 g powdered gamma alumina (Harshaw 3438). The vessel was fitted with a stirrer and a Dean Stark water trap. Over a four hour period the mixture was heated to 180° C. It was then stirred at 180-190° C for three hours. Sample 1 was taken at two hours and Sample 2 at three hours. The mixture was stirred at 180-190° C for five more hours and then Sample 3 was taken. Following are the results:

| Sample | Reaction Time | Product Composition (area percent) 2-benzylphenol | 2,6-dibenzylphenol |
|---|---|---|---|
| 1 | 2 hrs. | 21.3 | 8.6 |
| 2 | 3 hrs. | 25.0 | 25.7 |
| 3 | 8 hrs. | 42.3 | 52.9 |

The results show that the reaction is very slow in the liquid phase. Only 52.9 area percent (by Gas Chromatograph) 2,6-dibenzylphenol had formed after 8 hours reaction.

The compounds made by the present process are useful in providing antioxidant protection in a broad range of organic materials of the type normally subject to oxidative deterioration in the presence of oxygen during use over an extended period. In other words, the organic compositions protected by the present antioxidants are the type in which the art recognizes the need for antioxidant protection and to which an

antioxidant of some type is customarily added to obtain an extended service life. The oxidative degradation protected against is the slow gradual deterioration of the organic composition rather than, for example, combustion. In other words, the present additives are not flame retarding additives nor flame suppressing additives and the degradation protected against is not combustion, but rather the gradual deterioration of the organic commposition due to the effects of oxygen over an extended period of time.

The novel compounds of the present invention are suitable for use in protecting organic material normally susceptible to gradual degradation due to the effects of oxygen.

Preferred as antioxidants of the invention are 2,4,6-tribenzylphenol the 2,6-dibenzyl-4-cycloalkylphenols. These compounds include 2,6-dibenzyl-4-cyclohexylphenol.

Also preferred are the 2,6-dibenzylphenols having a 4 substituent which is an alkoxy. These compounds include 2,6-dibenzyl-4-methoxyphenol, 2,6-dibenzyl-4-ethoxyphenol, 2,6-dibenzyl-4-isopropoxyphenol, and 2,6-dibenzyl-butoxyphenol.

Examples of organic materials in which the additives are useful include polyolefins such a polyethylene, polypropylene, polybutadiene, and the like. Copolymers of olefinically unsaturated monomers such as styrene-butadiene rubber (SBR rubber), ethylene-propylene-diene terpolymers such as the terpolymer of ethylene, propylene and cyclopentadiene or cyclooctadiene, likewise, acrylonitrile butadiene-styrene resins are effectively stabilized. Ethylene-vinyl acetate copolymers are protected, as are butene methylacrylate copolymers. Nitrogen-containing polymers such as polyurethanes, nitrile rubber, and lauryl acrylate-vinylpyrolidone copolymers are effectively stabilized. Adhesive compositions such as solutions of polychloroprene (neoprene) in toluene are protected. Fats and oils of animal and vegetable origin are protected against gradual deterioration. Examples of these are lard, beef tallow, coconut oil, safflower oil, castor oil, babassu oil, cottonseed oil, corn oil, and rapeseed oil.

Petroleum oils and waxes such as solvent-refined, midcontinent lubricating oils are effectively stabilized. Animal feeds such as ground corn, cracked wheat, oats, wheat germ, alfalfa, and the like, are protected by mixing a small but effective amount of the present additive with these products. Vitamin extracts, especially the fat-soluble vitamins such as Vitamin A, B, D and C, are effectively stabilized against degradation. The additives are useful in foamed plastics such as expanded polystyrene, polyurethane foams, and the various foamed rubbers, alkyd resins such as short oil terephthalic acid-glycerol-linseed oil resins, and typical long oil resins of trimellitic acid-glycol-tung oil resins including epoxide-modified alkyl resins. Epoxy resins themselves such as isopropylidenebisphenol-epichlorohydrin epoxy resins are stabilized against degradation.

Hydrocarbons such as gasoline, kerosene, diesel fuel, fuel oil, furnace oil, and jet fuel are effectively protected. Likewise, synthetic hydrocarbon lubricants, for example, alphadecene trimer, polybutene lubricants, di- and tri- $C_{12-30}$ alkylated benzene and naphthalene synthetic lubricants are likewise protected.

Organometallics such as tetraethyllead, tetramethyllead, tetravinyllead, ferrocene, methyl ferrocene, cyclopentadienyl manganese tricarbonyl, methyl cyclopentadienyl manganese tri carbonyl, and cyclopentadienyl nickel nitrosyl, are effectively protected against oxidative degradation. Silicone oils and greases are also protected.

Synthetic ester lubricants such as those used in turbines and turbojet engines are given a high degree of stabilization. Typical synthetic ester lubricants include di-2-ethylhexyl sebacate, trimethylolpropane tripelargonate, $C_{5-9}$ aliphatic monocarbonxylic esters of pentaerythritol, complex esters formed by condensing under esterifying conditions, mixtures of polyols, polycarbonxylic acids, and aliphatic monocarboxylic acids and/or monohydric alkanols. An example of these complex esters is the condensation product formed from adipic acid, ethyleneglycol and amixture of $C_{5-9}$ aliphatic monocarboxylic acids. Plasticizers such as dioctyl phthalate are effectively protected. Heavy petroleum fractions such as tar and asphalt can also be protected should the need arise.

Polyamides such as adipic acid-1,6-diaminohexane condensates, and poly-6-aminohexanoic acid (nylon) are effectively stabilized. Polyalkylene oxides such as copolymers of phenol with ethylene oxide or propylene oxide are stabilized. Polyphenyl ethers such as poly-2,6-dimethylphenyl ether formed by polymerization of 2,6-dimethylphenol using a copper-pyridine catalyst are stabilized. Polycarbonate plastics and other polyformaldehydes are also protected.

Linear polyesters such as phthalic anhydride-glycol condensates are given a high degree of protection. Other polyesters such as trimellitic acid-glycerol condensates are also protected. Polyacrylates such as polymethylacrylate and polymethylmethacrylate are effectively stabilized. Polyacrylonitriles and copolymers of acylonitriles with other olefinically unsaturated monomers such as methylmethacrylates are also effectively stabilized.

The additives can be used to protect any of the many organic substrates to which an antioxidant is normally added. It can be used where economics permit to protect such substrates as road tar, paper,

polyvinyl acetate, coumarone-indene resins, polyvinyl ethers, polyvinylidene bromide, acrylonitrile, vinyl bromide copolymer, vinyl butyral resins, silicones such as dimethylsilicone lubricants, phosphate lubricants such as tricresylphosphate.

The additives are incorporated into the organic substrate in a small but effective amount so as to provide the required anticxidant protection. A useful range is from 0.01 to 5 weight percent, and a preferred range is from about 0.1 to 3 weight percent.

The additives can be used alone or together with a synergist. Exceptionally effective synergists, especially in homopolymers and copolymers of ethylenically unsaturated monomers, are the di-$C_{4-30}$ alkyl thiodipropionates such as dilauryl thiodipropionate and distearyl thiodipropionate. A useful range for such synergists is 0.01-5 weight percent and a more preferred range is 0.1-3 weight percent.

Methods of incorporating the additive into the substrate are well known. For example, if the substrate is liquid the additive can be merely mixed into the substrate. Frequently, the organic substrate is in solution and the additive is added to the solution and the solvent removed. Solid organic substrates can be merely sprayed with a solution of the additive in a volatile solvent. For example, stabilized grain products result from spraying the grain with a toluene solution of the additive. In the case of rubbery polymers the additive can be added following the polymerization stage by mixing it with the final emulsion or solution polymerization mixture and then coagulating or removing solvent to recover the stabilized polymer. It can also be added at the compounding stage by merely mixing equipment such as a Banbury blender. In this manner, rubbery polymers such as styrene-butadiene rubber, cis-polybutadiene or isoprene polymers are blended with the antioxidant together with the other ingredients normally added such as carbon black, oil, sulfur, zinc oxide, stearic acid, vulcanization accelerators, and the like. Following mastication, the resultant mixture is fabricated and molded into a finished form and vulcanized. The following will serve to illustrate the manner in which the additives are blended with various organic substrates.

EXAMPLE 11

A butadiene-acrylonitrile copolymer is prepared from 1,3-butadiene and 32 percent of acrylonitrile. One percent, based on the weight of polymer, 2,6-dibenzyl-4-methoxyphenol is added as an emulsion in a sodium oleate solutions. The latex is coagulated and the coagulum is washed and dried, resulting in a stabilized butadiene-acrylonitrile copolymer.

## Claims

1. A process for the preparation of a di-ortho benzyl substituted phenol comprising contacting a mixture of a phenol and a benzyl alcohol in the vapor phase with an activated gamma-alumina catalyst at a temperature which is from 225-450° C and is high enough to maintain said phenol and benzyl alcohol in the vapor phase, said phenol having at least both positions ortho to its phenolic hydroxyl group unsubstituted (except for hydrogen), and said mixture having a molar ratio of 1:1-3 of said phenol to said benzyl alcohol.

2. A process as claimed in claim 1, wherein the temperature is 250-350° C.

3. A process as claimed in claim 1 or claim 2, wherein said phenol has the structure

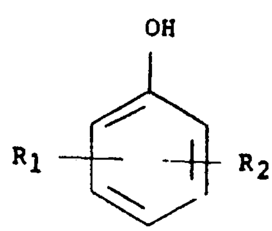

$R_1$ and $R_2$ each being independently hydrogen, alkyl containing 1-20 carbon atoms, alkenyl containing 2-20 carbon atoms, cycloalkyl containing 5-8 carbon atoms, aryl containing 6-12 carbon atoms, halogen, hydroxy or $C_{1-4}$ alkoxy.

4. A process as claimed in claim 1 which process comprises contacting a mixture of said phenol and said benzyl alcohol in the mole ratio of 1:1.5-2.5 at a temperature which is from 225-450 °C, preferably 250-350 °C.

5. A process as claimed in claim 4, wherein said phenol is the compound phenol and 2,6-dibenzylphenol is recovered as the major product.

6. A compound preparable by a process as claimed in claim 1 and having the structure

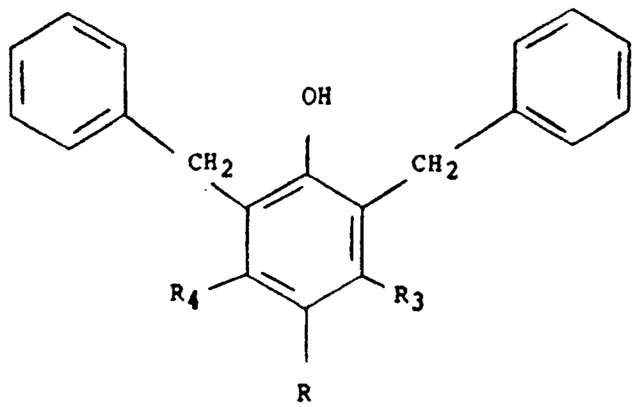

wherein $R_3$ and $R_4$ are each independently H, alkyl, cycloalkyl, halo, aralkyl, alkaryl, aryl, alkoxy, or alkenyl, and R is benzyl, cycloalkyl, hydroxy, alkoxy, alkenyl, or hydrocarbonyl.

7. 2,4,6-Tribenzylphenol.

8. 2,6-Dibenzyl-4-methoxyphenol.

9. 2,6-Dibenzyl-4-ethoxyphenol.

10. Organic material normally susceptible to degradation due to the effects of oxygen and containing as an antioxidant a compound as claimed in any one of claims 6 to 9.

11. The use as an antioxidant of a compound as claimed in any one of claims 6 to 9.

12. The modification of a process as claimed in any one of claims 1 to 5 characterised in that a di-ortho benzyl substituted phenol is prepared which thereafter is employed as an antioxidant.

**Revendications**

1. Procédé de préparation d'un phénol di-ortho-substitué par benzyle, procédé comprenant la mise en contact d'un mélange en phase vapeur d'un phénol et d'un alcool benzylique avec un catalyseur constitué d'une gamma-alumine activée, à une température de 225 - 450 °C, suffisante pour maintenir les dits phénol et alcool benzylique en phase vapeur, au moins les deux positions du dit phénol ortho par rapport à son groupe phénolique hydroxyle étant non substituées (sauf par l'hydrogène), et le dit mélange ayant un rapport molaire de 1 : 1-3 du dit phénol au dit alcool benzylique.

2. Procédé conforme à la revendication 1, dans lequel la température est située entre 250 et 350 °C.

3. Procédé conforme à l'une des revendications 1 ou 2, dans lequel le dit phénol possède la structure

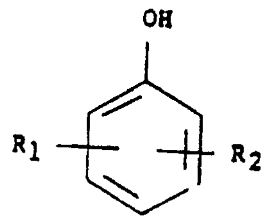

$R_1$ et $R_2$ étant chacun et de manière indépendante l'hydrogène, un alkyle contenant de 1 - 20 atomes de carbone, un alcényle contenant 2 - 20 atomes de carbone, un cycloalkyle contenant 5 - 8 atomes de carbone, un aryle contenant 6 - 12 atomes de carbone, un halogène, un groupe hydroxy ou un alkoxy en $C_{1-4}$.

4. Procédé conforme à la revendication 1, procédé comprenant la mise en contact d'un mélange du dit phénol et du dit alcool benzylique dans le rapport molaire de 1 : 1,5-2,5 à une température située entre 225 - 450° C, de préférence 250 - 350° C.

5. Procédé conforme à la revendication 4, dans lequel le dit phénol est le composé phénol, et le 2,6-dibenzylphénol se retrouve comme produit principal.

6. Un composé susceptible d'être obtenu par un procédé conforme à la revendication 1 et possédant la structure

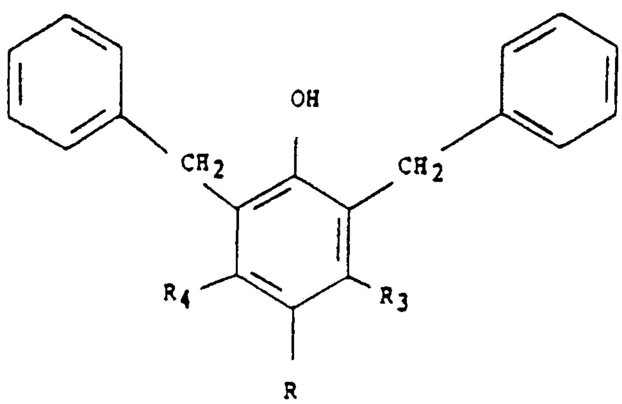

dans laquelle $R_3$ et $R_4$ sont chacun et de manière indépendante H, un alkyle, un cycloalkyle, un halogène, un aralkyle, un alkaryle, un aryle, un alkoxy ou un alcényle, et où R est un benzyle, un cycloalkyle, un hydroxy, un alkoxy, un alcényle ou un hydrocarbonyle.

7. 2,4,6-tribenzylphénol.

8. 2,6-dibenzyl-4-méthoxyphénol.

9. 2,6-dibenzyl-4-éthoxyphénol.

10. Matériau organique normalement susceptible d'une dégradation due aux effets de l'oxygène et contenant à titre d'antioxydant un composé conforme à l'une des revendications 6 à 9.

11. Utilisation comme antioxydant d'un composé conforme à une quelconque des revendications 6 à 9.

12. Modification d'un procédé conforme à une quelconque des revendications 1 à 5, caractérisée en ce qu'un phénol di-ortho-substitué par un benzyle est préparé pour être ensuite utilisé comme antioxydant.

**Ansprüche**

1. Verfahren zur Herstellung eines zweifach in ortho-Stellung Benzyl-substituierten Phenols, welches das In-Kontakt-Bringen einer Mischung eines Phenols und eines Benzylalkohols in der Dampfphase mit einem aktivierten gamma-Aluminiumoxid-Katalysator bei einer Temperatur umfaßt, die im Bereich von 225 bis 450 °C liegt und hoch genug ist, das Phenol und den Benzylalkohol in der Dampfphase zu halten, wobei das Phenol wenigstens an den beiden Positionen, die in ortho-Stellung zu seiner phenolischen Hydroxylgruppe stehen, keinen Substituenten außer Wasserstoff trägt, und wobei die Mischung ein Molverhältnis Phenol : Benzylalkohol von 1 : 1 bis 3 aufweist.

2. Verfahren nach Anspruch 1, worin die Temperatur bei 250 bis 350 °C liegt.

3. Verfahren nach Anspruch 1 oder Anspruch 2, worin das Phenol die Struktur

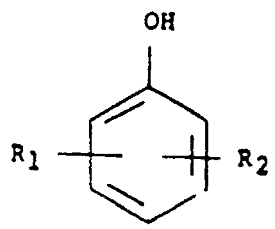

hat, worin $R_1$ und $R_2$ jeweils unabhängig voneinander Wasserstoff, 1 bis 20 Kohlenstoffatome enthaltende Alkylreste, 2 bis 20 Kohlenstoffatome enthaltende Alkenylreste, 5 bis 8 Kohlenstoffatome enthaltende Cycloalkylreste, 6 bis 12 Kohlenstoffatome enthaltende Arylreste, Halogen, Hydroxy oder $C_{1-4}$-Alkoxy sind.

4. Verfahren nach Anspruch 1, welches das In-Kontakt-Bringen einer Mischung des Phenols und des Benzylalkohols im Molverhältnis 1 : 1,5 bis 2,5 bei einer Temperatur umfaßt, die im Bereich von 225 bis 450 °C, vorzugsweise bei 250 bis 350 °C liegt.

5. Verfahren nach Anspruch 4, worin das Phenol die Verbindung Phenol ist und 2,6-Dibenzylphenol als das Hauptprodukt gewonnen wird.

6. Nach einem Verfahren gemäß Anspruch 1 herstellbare Verbindung mit der Struktur

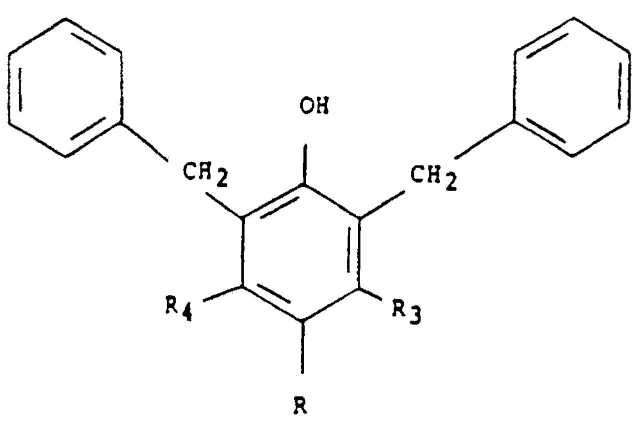

worin $R_3$ und $R_4$ jeweils unabhängig voneinander H, Alkyl,Cycloalkyl, Halogen, Aralkyl, Alkaryl, Aryl, Alkoxy oder Alkenyl sind und R Benzyl, Cycloalkyl, Hydroxy, Alkoxy, Alkenyl oder Hydrocarbonyl ist.

7. 2,4,6-Tribenzylphenol.

8. 2,6-Dibenzyl-4-methoxyphenol.

9. 2,6-Dibenzyl-4-ethoxyphenol.

10. Organisches Material, das normalerweise empfindlich gegenüber einem Abbau aufgrund der Einwirkungen von Sauerstoff ist und als Antioxidationsmittel eine Verbindung gemäß irgendeinem der Ansprüche 6 bis 9 enthält.

11. Verwendung einer Verbindung nach irgendeinem der Ansprüche 6 bis 9 als Antioxidationsmittel.

12. Abwandlung eines Verfahrens nach irgendeinem der Ansprüche 1 bis 5, dadurch gekennzeichnet, daß ein zweifach in ortho-Stellung Benzyl-substituiertes Phenol hergestellt wird, das danach als Antioxidationsmittel eingesetzt wird.